(19) Europäisches Patentamt
European Patent Office
Office européen des brevets

(11) Veröffentlichungsnummer : **0 102 578**
**B1**

(12) # EUROPÄISCHE PATENTSCHRIFT

(45) Veröffentlichungstag der Patentschrift : **30.07.86**

(51) Int. Cl.⁴ : **C 07 D405/06**, A 01 N 43/653, A 01 N 43/50// C07D321/06

(21) Anmeldenummer : **83108254.0**

(22) Anmeldetag : **22.08.83**

(54) **2-Aryl-2-azolylmethyl-1,3-dioxepine.**

(30) Priorität : **03.09.82 DE 3232737**

(43) Veröffentlichungstag der Anmeldung : **14.03.84 Patentblatt 84/11**

(45) Bekanntmachung des Hinweises auf die Patenterteilung : **30.07.86 Patentblatt 86/31**

(84) Benannte Vertragsstaaten : **AT BE CH DE FR GB IT LI NL SE**

(56) Entgegenhaltungen :
**DE-A- 2 431 407**
**DE-A- 2 551 560**
**US-A- 3 575 999**

(73) Patentinhaber : **BAYER AG**
**Konzernverwaltung RP Patentabteilung**
**D-5090 Leverkusen 1 Bayerwerk (DE)**

(72) Erfinder : **Kraatz, Udo, Dr.**
**Andreasstrasse 22a**
**D-5090 Leverkusen 1 (DE)**
Erfinder : **Jäger, Gerhard, Dr.**
**Gellertstrasse 18**
**D-5090 Leverkusen 1 (DE)**
Erfinder : **Büchel, Karl Heinz, Prof., Dr.**
**Dabringhausener Strasse 42**
**D-5093 Burscheid (DE)**
Erfinder : **Brandes, Wilhelm, Dr.**
**Eichendorffstrasse 3**
**D-5653 Leichlingen (DE)**
Erfinder : **Reinecke, Paul, Dr.**
**Lessingstrasse 11**
**D-5090 Leverkusen 3 (DE)**
Erfinder : **Scharf, Hans-Dieter, Professor Dr.**
**Greppstrasse 15a**
**D-5106 Roetgen (DE)**
Erfinder : **Frauenrath, Herbert, Dr.**
**Breitbendenstrasse 96**
**D-5100 Aachen (DE)**

**EP 0 102 578 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 2-Aryl-2-azolylmethyl-1,3-dioxepine, ein Verfahren zu ihrer Herstellung und ihre Verwendung als Pflanzenschutzmittel.

Es ist bereits bekannt geworden, daß bestimmte Azolylmethylketale wie z. B. 2-(2,4-Dichlorphenyl)-4-n-propyl-2-(1,2,4-triazol-1-yl-methyl)-1,3-dioxolan oder 2-(2,4-Dichlorphenyl)-4-methyl-2-(1,2,4-triazol-1-yl-methyl)-1,3-dioxolan (vergleiche DE-A-2 551 560) oder auch bestimmte Azolylmethylcarbinole wie z. B. (1,2,4-Triazol-1-yl-methyl)-(4-chlorphenyl)-carbinol (vergleiche DE-A-2 431 407 fungizide Eigenschaften besitzen.

Weiterhin ist schon bekannt, daß auch bestimmte Azol-Derivate, die einen 1,3-Dioxanring enthalten, fungizid wirksam sind (vgl. US-A-3 575 999).

Die Wirkung dieser Verbindungen ist jedoch insbesondere bei niedrigen Aufwandmengen und -konzentrationen nicht immer ganz zufriedenstellend.

Es wurden neue 2-Aryl-2-azolylmethyl-1,3-dioxepine der allgemeinen Formel (I),

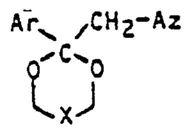

(I)

in welcher

Az für Imidazol-1-yl oder 1,2,4-Triazol-1-yl steht,

X für die Ethylen- oder die Vinylen-Gruppierung steht und

Ar für Phenyl steht, welches gegenbenenfalls substituiert sein kann durch Halogen, Cyano, Nitro, Alkyl, Alkoxy und Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, Dialkylamino, Alkoxycarbonyl, Alkoxycarbonylamino und N-Alkyl-alkoxycarbonylamino mit jeweils 1 bis 4 Kohlenstoffatomen in den jeweiligen Alkylteilen, sowie durch gegebenenfalls durch Halogen oder Alkyl mit 1 bis 4 Kohlenstoffatomen substituiertes Phenyl oder Phenoxy,

sowie deren pflanzenverträgliche Säureadditions-Salze und Metallsalz-Komplexe gefunden.

Weiterhin wurde gefunden, daß man die neuen 2-Aryl-2-azolylmethyl-1,3-dioxepine der allgemeinen Formel (I) sowie deren pflanzenverträgliche Säureadditions-Salze und Metallsalz-Komplexe erhält, wenn man die entsprechenden 2-Aryl-2-halogenmethyl-1,3-dioxepine der Formel (II)

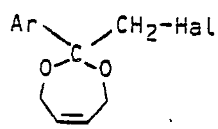

(II)

in welcher

Ar die oben angegebene Bedeutung hat und

Hal für Halogen steht,

mit Alkalisalzen von Azolen der Formel (III),

$$Az—M \qquad (III)$$

in welcher

Az die oben angegebene Bedeutung hat und

M für ein Alkalimetall steht,

in Gegenwart eines Verdünnungsmittels umsetzt zu den erfindungsgemäßen Verbindungen der Formel (Ia),

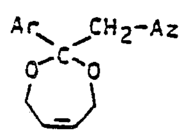

(Ia)

in welcher Ar und Az die oben angegebene Bedeutung haben, und

diese dann gegebenenfalls mit Wasserstoff in Gegenwart eines Katalysators und gegebenenfalls in Gegenwart eines Verdünnungsmittels zu den erfindungsgemäßen Verbindungen der Formel (Ib),

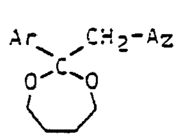

(Ib)

in welcher Ar und Az die oben angegebene Bedeutung haben, katalytisch hydriert und an die so erhaltenen Verbindungen der Formel (Ia) oder (Ib) noch gegebenenfalls eine Säure oder ein Metallsalz addiert.

Die neuen 2-Aryl-2-azolylmethyl-1,3-dioxepine der Formel (I) weisen starke fungizide Eigenschaften auf. Dabei zeigen die erfindungsgemäßen Verbindungen überraschenderweise eine bessere fungizide Wirksamkeit als die nach dem Stand der Technik bekannten Verbindungen 2-(2,4-Dichlorphenyl)-4-n-propyl-2-(1,2,4-triazol-1-yl-methyl)-1,3-dioxolan oder 2-(2,4-Dichlorphenyl)-4-methyl-2-(1,2,4-triazol-1-yl-methyl)-1,3-dioxolan oder (1,2,4-Triazol-1-yl-methyl)-(4-chlorphenyl)-carbinol, welches chemisch und wirkungsmäßig naheliegende Verbindungen sind. Außerdem übertreffen die erfindungsgemäßen Stoffe unerwarteterweise auch die aus der US-A-3 575 999 bekannten Verbindungen bezüglich ihrer fungiziden Eigenschaften.

Die erfindungsgemäßen 2-Aryl-2-azolylmethyl-1,3-dioxepine sind durch die Formel (I) allgemein definiert. Sie unterscheiden sich von den aus der DE-A-2 551 560 und der US-A-3 575 999 vorbekannten Stoffen dadurch, daß sie anstatt eines 1,3-Dioxolan- bzw. 1,3-Dioxan-Ringes jeweils ein 1,3-Dioxepin-System enthalten.

Bevorzugt sind diejenigen Verbindungen der Formel (I), in denen

Az und X die in der Erfindungsdefinition angegebene Bedeutung haben und

Ar für gegebenenfalls ein- bis dreifach gleich oder verschieden substituiertes Phenyl steht, wobei als Substituenten ganz besonders bevorzugt sind : Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethoxycarbonyl, Dimethylamino, Acetamido, N-Methylacetamido, sowie durch Fluor, Chlor oder Methyl, ein- bis dreifach gleich oder verschieden substituiertes Phenyl oder Phenoxy.

Verwendet man beispielsweise 2-Brommethyl-2-(4-chlorphenyl)-1,3-2H-4,7-dihydrodioxepin und das Natriumsalz des Imidazols als Ausgangsstoffe, so kann der Reaktionsablauf durch das folgende Formelschema wiedergegeben werden :

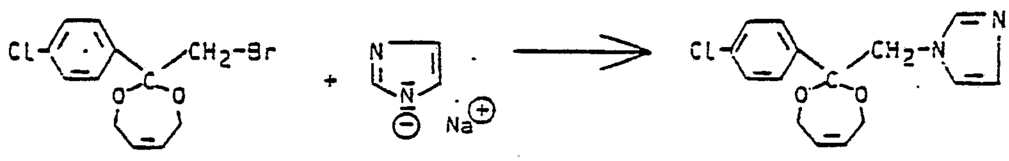

Verwendet man beispielsweise 2-(4-Chlorphenyl)-2-(imidazol-1-yl-methyl)-1,3-2H-4,7-dihydrodioxepin als Ausgangsstoff und Wasserstoff als Hydrierungsmittel, so kann der Reaktionsablauf durch das folgende Formelschema wiedergegeben werden :

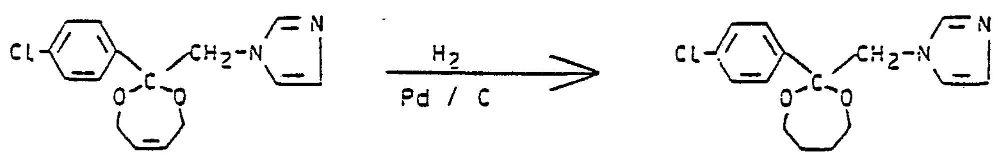


Die bei der Durchführung des erfindungsgemäßen Verfahrens als Ausgangsstoffe benötigten 2-Aryl-2-halogenmethyl-1,3-dioxepine sind durch die Formel (II) allgemein definiert.

In dieser Formel (II) besitzt Ar vorzugsweise dieselbe Bedeutung, die bei der Beschreibung der erfindungsgemäßen Stoffe der Formel (I) vorzugsweise für diesen Substituenten genannt wurde. Hal steht vorzugsweise für Chlor oder Brom.

Die 2-Aryl-2-halogenmethyl-1,3-dioxepine der Formel (II) sind noch nicht bekannt. Man erhält sie, indem man die entsprechenden Aracylhalogenide der allgemeinen Formel (IV),

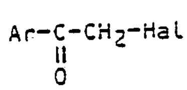
(IV)

in welcher Ar und Hal die oben angegebene Bedeutung haben, zunächst mit Ameisensäuretrimethylorthoester oder Acetondimethylacetal in Gegenwart eines Verdünnungsmittels, wie z. B. Methanol, und in Gegenwart eines sauren Katalysators, wie z. B. p-Toluolsulfonsäure, bei Temperaturen zwischen 40 °C und 100 °C ketalisiert und in einer zweiten Stufe die so erhaltenen Dimethylketale der Formel (V)

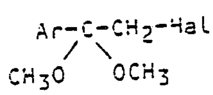
(V)

in welcher Ar und Hal die oben angegebene Bedeutung haben, mit 2-Buten-1,4-diol in Gegenwart eines sauren Katalysators, wie z. B. o-Toluolsulfonsäure, bei Temperaturen zwischen 20 °C und 80 °C gegebenenfalls unter vermindertem Druck umketalisiert.

Die Aracylhalogenide der Formel (IV) sind bekannt (vergleiche J. Amer. Chem. Soc. *52*, 818 (1930) ; US-A-3 936 470 ; Zh. Obshch. Khim. *33*, 1135 (1963)).

Die außerdem für das erfindungsgemäße Verfahren als Ausgangsstoffe zu verwendenden Alkalisalze von Azolen sind durch die Formel (III) allgemein definiert. In dieser Formel hat Az vorzugsweise die in der allgemeinen Erfindungsdefinition angegebene Bedeutung. M steht vorzugsweise für Natrium oder Kalium.

Die Alkalisalze von Azolen der Formel (III) sind allgemein bekannt. Sie werden durch Umsetzung von Imidazol bzw. 1,2,4-Triazol mit Natrium- oder Kaliummethylat oder durch Umsetzung von Imidazol bzw. Triazol mit der äquivalenten Menge des entsprechenden Alkalihydrids erhalten.

Als Verdünnungsmittel kommen für das erfindungsgemäße Verfahren inerte organische Lösungsmittel infrage. Hierzu gehören vorzugsweise Amide, wie Dimethylformamid oder Dimethylacetamid oder N-Methylacetanilid, ferner Dimethylsulfoxid oder Hexamethylphosphorsäuretriamid.

Als Verdünnungsmittel für die Hydrierung der erfindungsgemäßen Substanzen (Ia) zu den erfindungsgemäßen Substanzen (Ib) kommen ebenfalls inerte organische Lösungsmittel infrage. Hierzu gehören vorzugsweise aliphatische oder aromatische Kohlenwasserstoffe, wie Benzin, Benzol, Toluol oder Xylol ; halogenierte Kohlenwasserstoffe, wie Methylenchlorid, Chloroform, Tetrachlorkohlenstoff oder Chlorbenzol ; Ether, wie Diethylether, Dioxan oder Tetrahydrofuran ; Ester wie Ethylacetat ; oder Alkohole, wie Methanol, Ethanol oder Isopropanol.

Bei der Durchführung der Hydrierung der erfindungsgemäßen Substanzen (Ia) zu den erfindungsgemäßen Substanzen (Ib), können alle üblichen Hydrierkatalysatoren eingesetzt werden. Vorzugsweise verwendet man Edelmetallkatalysatoren, wie z. B. Platin oder Palladium, gegebenenfalls auf einem geeigneten Trägerstoff, wie z. B. Kohle.

Die Reaktionstemperaturen können bei der Durchführung des erfindungsgemäßen Verfahrens in einem größeren Bereich variiert werden. Im allgemeinen arbeitet man zwischen + 20 °C und + 180 °C, vorzugsweise zwischen + 60 °C und + 150 °C.

Die Reaktionstemperatur bei der Hydrierung der erfindungsgemäßen Substanzen (Ia) zu den erfindungsgemäßen Substanzen (Ib) kann ebenfalls in einem größeren Bereich variiert werden. Im allgemeinen arbeitet man zwischen + 10 °C und + 200 °C, vorzugsweise zwischen + 20 °C und + 100 °C.

Die Hydrierung der erfindungsgemäßen Substanzen (Ia) zu den erfindungsgemäßen Substanzen (Ib) kann bei erhöhtem Druck durchgeführt werden. Im allgemeinen arbeitet man in Druckbereichen zwischen 1 und 10 atm, vorzugsweise zwischen 4 und 6 atm.

Bei der Durchführung des erfindungsgemäßen Verfahrens setzt man auf 1 Mol der Verbindung der Formel (II) vorzugsweise 1 bis 2 Mol des Azol-Alkalisalzes der Formel (III) ein. Die Isolierung der Verbindungen der Formel (Ia) erfolgt nach üblichen Methoden.

Bei der Durchführung der Hydrierung der erfindungsgemäßen Substanzen (Ia) zu den erfindungsgemäßen Substanzen (Ib) setzt man auf 0,1 Mol Verbindung der Formel (Ia) 1 bis 5 g, vorzugsweise 2 bis 3 g Hydrierkatalysator zu. Die Hydrierung und Aufarbeitung sowie die Isolierung der Verbindungen der Formel (Ib) erfolgt nach üblichen Methoden.

Zur Herstellung von physiologisch verträglichen Säureadditionssalzen der Verbindungen der Formel (I) kommen vorzugsweise folgende Säuren infrage : Halogenwasserstoffsäuren, wie z. B. Chlorwasserstoffsäure und Bromwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z. B. durch Lösen einer Verbindung der Formel (I) in einem geeigneten inerten Lösungsmittel und Hinzufügen der Säure, wie z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten : Halogenwasserstoffsäuren, wie z. B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten

notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mt besonders gutem Erfolg zur Bekämpfung von Venturia-Arten, wie z. B. gegen den Erreger des Apfelschorfs (Venturia inaequalis), von Erysiphe-Arten, wie z. B. gegen den Erreger des echten Getreidemehltaus (Erysiphe graminis) oder von Sphaerotheca-Arten, wie z. B. gegen den Erreger des echten Gurkenmehltaus (Sphaerotheca fuliginea) eingesetzt werden. Daneben zeigen die erfindungsgemäßen Verbindungen auch eine gute Wirkung gegen die Getreidekrankheiten Rost, Septoria nodorum, Cochliobolus sativus und Pyrenophora teres sowie gegen die Reiskrankheiten Pyricularia oryzae und Pellicularia sasakii.

In höheren Aufwandmengen zeigen die erfindungsgemäßen Stoffe auch eine selektiv-herbizide und eine wachstumsregulierende Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfs lösungsmittel verwendet werden. Als flüssige Lösungsmittel kommen im wesentlichen infrage : Aromaten, wie Xylol, Toluol, oder Alkyl-naphthaline, chlorierte Aromaten oder chlorierte aliphatische Kohlenwasserstoffe, we Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage ; z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln ; als Emulgier- und/oder schaumerzeugende Mittel kommen in Frage : z. B. nichtionogene und anionische Emulgatoren, wie Polyoxyethylen-Fettsäureester, Polyoxyethylen-Fettalkohol-ether, z. B. Alkylarylpolyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,0000 1 bis 0,1 Gew.-%, vorzugsweise von 0,000 1 bis 0,02 Gew.-%, am Wirkungsort erforderlich.

## Herstellungsbeispiele

### Beispiel 1

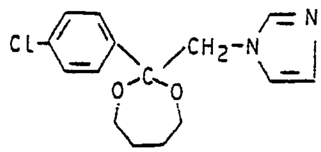

14 g (0,2 Mol) 1,2,4-Triazol werden in 100 ml Dimethylformamid bei 20 bis 25 °C portionsweise mit 6 g (0,2 Mol) 80 %-igem Natriumhydrid versetzt. Nach 10 Minuten gibt man tropfenweise 30,1 g (0,1 Mol) 2-Brommethyl-2-(4-chlorphenyl)-1,2-2H-4,7-dihydrodioxepin in 75 ml Dimethylformamid zu und rührt 8 Stunden bei 140 °C. Man destilliert die Hauptmenge Dimethylformamid im Vakuum ab und nimmt den Rückstand in Wasser auf. Die Mischung wird dreimal mit Methylenchlorid extrahiert und die vereinigten Methylenchloridextratke werden mit Wasser gewaschen und im Vakuum eingeengt und zur Reinigung über eine kurze Kieselgelsäule filtriert. Nach Abdampfen des Lösungsmittels erhält man 21 g (72 % der Theorie) an 2-(4-Chlorphenyl)-2-(1,2,4-triazol-1-yl-methyl)-1,3-2H-4,7-dihydrodioxepin vom Schmelzpunkt 100 °C.

### Beispiel 2

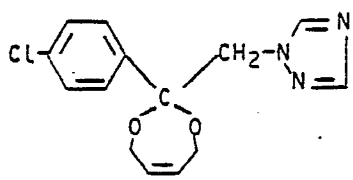

25 g (0,09 Mol) an 2-(4-Chlorphenyl)-2-(1,2,4-imidazol-1-yl-methyl)-1,3-2H-4,7-dihydrodioxepin werden in 300 ml Ethylacetat gelöst und mit 3 g Palladium auf Kohle bei 4 bis 6 atm und 20 °C-25 °C hydriert. Nach beendeter Wasserstoffaufnahme filtriert man den Katalysator ab und entfernt das Lösungsmittel im Vakuum. Der ölige Rückstand kristallisiert beim Verrühren mit Cyclohexan. Nach Absaugen und Trocknen der Kristalle erhält man 19 g (75 % der Theorie) an 2-(4-Chlorphenyl)-2-(1,2,4-imidazol-1-yl-methyl)-1,3-perhydrodioxepin vom Schmelzpunkt 65 °C bis 68 °C.

Entsprechend den Beispielen 1 und 2 sowie entsprechend der angegebenen Verfahrensbedingungen können die folgenden Verbindungen der allgemeinen Formel (I)

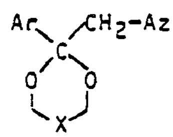 (I)

erhalten werden.

| Beisp.-Nr. | Ar | Az | X | Schmelz-punkt(°C) |
|---|---|---|---|---|
| 3 | 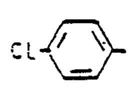 | 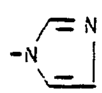 | -CH=CH- | 151 |
| 4 | 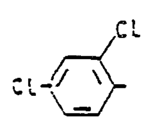 | 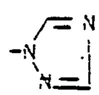 | -CH=CH- | 82 |
| 5 | 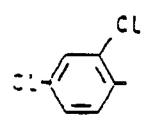 | 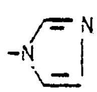 | -CH=CH- | 107 |
| 6 | 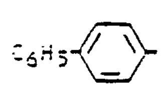 | 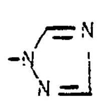 | -CH=CH- | 129 |

Fortsetzung

| Beisp.-Nr. | Ar | Az | X | Schmelzpunkt (°C) |
|---|---|---|---|---|
| 7 | 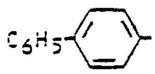 | 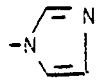 | $-CH=CH-$ | 130 |
| 8 | 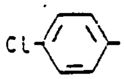 | 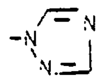 | $-CH_2-CH_2-$ | 85 |
| 9 | 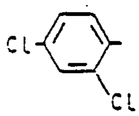 | 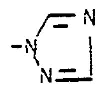 | $-CH_2-CH_2-$ | 60 |
| 10 | 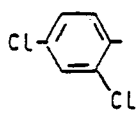 | 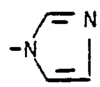 | $-CH_2-CH_2-$ | 55 - 60 |
| 11 | 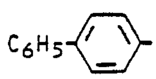 | 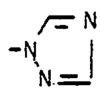 | $-CH_2-CH_2-$ | 97 |
| 12 | 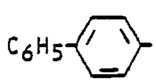 | 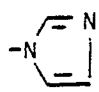 | $-CH_2-CH_2-$ | 97 |

Herstellung der Ausgangsprodukte der Formel (II):

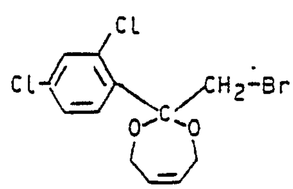

(II-1)

Man mischt 666, 6 g (2,1 Mol) 2,4-Dichlorphenacylbromid-dimethylketal mit 1 Liter 2-Buten-1,4-diol und 2 g Paratoluolsulfonsäure in einer Destillationsapparatur und erwärmt im Wasserstrahlvakuum unter Rühren 12 Stunden auf 40 °C. Nach dem Abkühlen wird das Reaktionsgemisch zwischen 1,5 Liter Ether und 2 Liter gesättigter Kaliumcarbonatlösung verteilt, die organische Phase abgetrennt und der ölige Rückstand nach Abdampfen des Lösungsmittels destilliert. Man erhält 326 g (46 % der Theorie) an 2-Brommethyl-2-(2,4-dichlorphenyl)-1,3-2H-4,7-dihydrodioxepin vom Siedepunkt $Kp_{0,25}$ : 150 °C.

In analoger Weise erhält man die folgenden Ausgangsprodukte der allgemeinen Formel (II) :

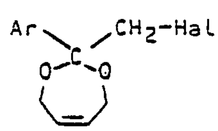

(II)

| Beisp.-Nr. | Ar | Hal | Schmelzpunkt (°C) |
|---|---|---|---|
| (II-2) | 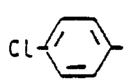 | Br | 74 |
| (II-3) | 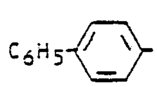 | Br | 107 |

Herstellung der Vorprodukte der Formel (V) :

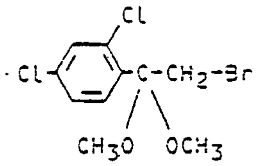

(V-I)

420 g (1,6 Mol) 2,4-Dichlorphenacylbromid werden mit 340 g (3,2 Mol) Orthoameisensäuretrimethylester und 5 g Paratoluolsulfonsäure in 500 ml Methanol gelöst und 12 Stunden am Rückfluß gekocht. Anschließend fügt man nochmals 100 g Orthoester und 2 g Paratoluolsulfonsäure zu und erhitzt weitere 10 Stunden am Rückfluß. Nach dem Abkühlen gießt man die Reaktionsmischung in gesättigte Kaliumcarbonatlösung und extrahiert das Produkt mit mehreren Portionen Methylenchlorid. Man wäscht die vereinigten Methylenchloridphasen mit Wasser, trocknet über Natriumsulfat und entfernt das Lösungsmittel im Vakuum. Der ölige Rückstand wird destilliert. Man erhält so 337 g (67 % der Theorie) an 2,4-Dichlorphenacylbromid-dimethylketal vom Siedepunkt $Kp_{0,15}$ : 135 °C.

In analoger Weise erhält man die folgenden Vorprodukte der allgemeinen Formel (V) :

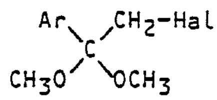

(V)

| Beisp.-Nr. | Ar | Hal | Schmelzpunkt (°C) |
|---|---|---|---|
| (V-2) | 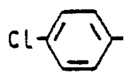 | Br | 50 |
| (V-3) | 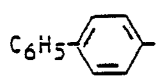 | Br | 94 |

## Anwendungsbeispiele

In den folgenden Anwendungsbeispielen werden die hier aufgeführten Verbindungen als Vergleichssubstanzen eingesetzt :

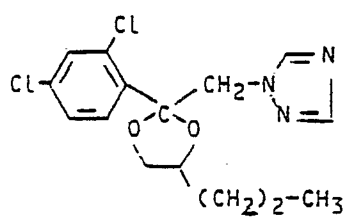

(A)

2-(2,4-Dichlorphenyl)-4-n-propyl-2-(1,2,4-triazol-1-yl-methyl)-1,3-dioxolan

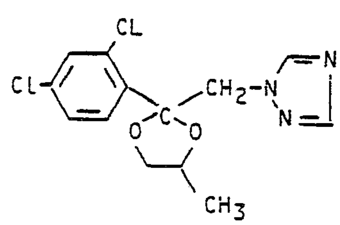

(B)

2-(2,4-Dichlorphenyl)-4-methyl-2-(1,2,4-triazol-1-yl-methyl)-1,3-dioxolan

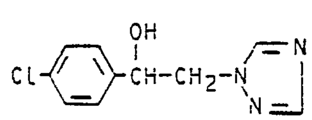

(C)

4-Chlorphenyl-(1,2,4-triazol-1-yl-methyl)-carbinol

## Beispiel A

Sphaerotheca-Test (Gurke)/protektiv

Lösungsmittel : 4,7 Gewichtsteile Aceton
Emulgator : 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : (4) und (9).

## Beispiel B

Venturia-Test (Apfel)/protektiv

Lösungsmittel : 4,7 Gewichtsteile Aceton
Emulgator : 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z. B. die Verbindung gemäß folgendem Herstellungsbeispiel : (9)

## Beispiel C

Erysiphe-Test (Gerste)/protektiv

Lösungsmittel : 100 Gewichtsteile Dimethylformamid
Emulgator : 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : (1), (3), (4), (5), (6), (2), (8), (11) und (12).

## Beispiel D

Erysiphe-Test (Gerste)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 × 12 Korn 2 cm tief in eine Standarderde. 7 Tage nach der Aussaat, wenn die jungen Pflanzen ihr erstes Blatt entfaltet haben, werden sit mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : (4) und (5).

**Patentansprüche**

1. 2-Aryl-2-azolylmethyl-1,3-dioxepine der allgemeinen Formel (I),

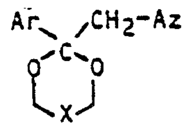

(I)

in welcher

Az für Imidazol-1-yl oder 1,2,4-Triazol-1-yl steht,

X für die Ethylen- oder die Vinylen-Gruppierung steht und

Ar für Phenyl steht, welches gegebenenfalls substituiert sein kann durch Halogen, Cyano, Nitro, Alkyl, Alkoxy und Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, Dialkylamino, Alkoxycarbonyl, Alkoxycarbonylamino und N-Alkyl-alkoxycarbonylamino mit jeweils 1 bis 4 Kohlenstoffatomen in den jeweiligen Alkylteilen, sowie durch gegebenenfalls durch Halogen oder Alkyl mit 1 bis 4 Kohlenstoffatomen substituiertes Phenyl oder Phenoxy,

sowie deren pflanzenverträgliche Säureadditions-Salze und Metallsalz-Komplexe.

2. Verbindungen der Formel (I) in Anspruch 1,

wobei

Ar für Phenyl steht, welches gegebenenfalls gleich oder verschieden ein- bis dreifach substituiert sein kann durch Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethoxycarbonyl, Dimethylamino, Acetamido, N-Methyl-acetamido, sowie durch gegebenenfalls durch Fluor, Chlor oder Methyl, ein- bis dreifach gleich oder verschieden substituiertes Phenyl oder Phenoxy, und

Az und X die in Anspruch 1 angegebenen Bedeutung haben, sowie der pflanzenverträgliche Säureadditionssalze und Metallsalz-Komplexe.

3. Verfahren zur Herstellung von 2-Aryl-2-azolylmethyl-1,3-dioxepinen der allgemeinen Formel (I),

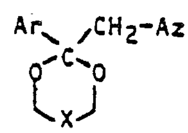

(I)

in welcher

Az für Imidazolyl-1-yl oder 1,2,4-Triazol-1-yl steht,

X für die Ethylen- oder Vinylen-Gruppierung steht und

Ar für Phenyl steht, welches gegebenenfalls substituiert sein kann durch Halogen, Cyano, Nitro, Alkyl, Alkoxy und Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, Dialkylamino, Alkoxycarbonyl, Alkoxycarbonylamino und N-Alkyl-alkoxycarbonylamino mit jeweils 1 bis 4 Kohlenstoffatomen in den jeweiligen Alkylteilen, sowie durch gegebenenfalls durch Halogen oder Alkyl mit 1 bis 4 Kohlenstoffatomen substituiertes Phenyl oder Phenoxy, sowie von deren pflanzenverträglichen Säureadditionssalzen und Metallsalz-Komplexen,

dadurch gekennzeichnet, daß man die entsprechenden 2-Aryl-2-halogenmethyl-1,3-dioxepine der Formel (II),

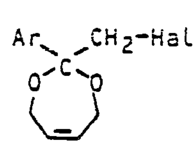

(II)

in welcher

Ar die oben angegebene Bedeutung hat und
Hal für Halogen steht,
mit Alkalisalzen von Azolen der Formel (III),

Az—M . (III)

in welcher
Az die oben angegebene Bedeutung hat und
M für ein Alkalimetall steht,
in Gegenwart eines Verdünnungsmittels umsetzt zu den erfindungsgemäßen Verbindungen der Formel (Ia),

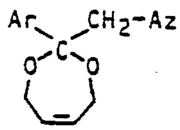
(Ia)

in welcher Ar und Az die oben angegebene Bedeutung haben, und
diese dann gegebenenfalls mit Wasserstoff in Gegenwart eines Katalysators und gegebenenfalls in Gegenwart eines Verdünnungsmittels zu den erfindungsgemäßen Verbindungen der Formel (Ib),

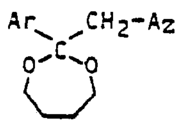
(Ib)

in welcher Ar und Az die oben angegebene Bedeutung haben,
katalytisch hydriert und an die so erhaltenen Verbindungen der Formel (Ia) oder (Ib) noch gegebenenfalls anschließend eine Säure oder ein Metallsalz addiert.

4. Fungizide Mittel, gekennzeichnet durch einen Gehalt an mindestens einem 2-Aryl-2-azolylmethyl-1,3-dioxepin der Formel (I) in Anspruch 1 und/oder einem seiner pflanzenverträglichen Säureadditionssalze und Metallsalz-Komplexe.

5. Verwendung von 2-Aryl-2-azolylmethyl-1,3-dioxepinen der Formel (I) in Anspruch 1 und/oder deren pflanzenverträglichen Säureadditionssalzen und Metallsalz-Komplexen als Pflanzenschutzmittel.

6. Verwendung von 2-Aryl-2-azolylmethyl-1,3-dioxepinen der Formel (I) in Anspruch 1 und/oder deren pflanzenverträglichen Säureadditionssalzen und Metallsalz-Komplexen zur Bekämpfung von Pilzen.

## Claims

1. 2-Aryl-2-azolylmethyl-1,3-dioxepines of the general formula (I)

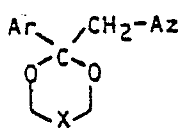
(I)

in which
Az represents imidazol-1-yl or 1,2,4-triazol-1-yl,
X represents the ethylene or the vinylene grouping and
Ar represents phenyl, which can be optionally substituted by halogen, cyano, nitro, alkyl, alkoxy and alkylthio with in each case 1 to 4 carbon atoms, halogenoalkyl, halogenoalkoxy and halogenoalkylthio with in each case 1 to 2 carbon atoms and 1 to 5 halogen atoms, dialkylamino, alkoxycarbonyl, alkoxycarbonylamino and N-alkyl-alkoxycarbonylamino with in each case 1 to 4 carbon atoms in the particular alkyl parts, and by phenyl or phenoxy which are optionally substituted by halogen or alkyl with 1 to 4 carbon atoms,
and their plant-tolerated acid addition salts and metal salt complexes.

2. Compounds of the formula (I) in Claim 1, wherein
Ar represents phenyl, which can be optionally mono- to tri-substituted by identical or different substituents from the group comprising fluorine, chlorine, bromine, cyano, nitro, methyl, methoxy,

methylthio, methoxycarbonyl, trifluoromethyl, trifluoromethoxy, trifluoromethylthio, ethyl, ethoxy, ethoxycarbonyl, dimethylamino, acetamido and N-methylacetamido, and by phenyl or phenoxy which are optionally mono- to tri-substituted by identical or different substituents from the group comprising fluorine, chlorine and methyl, and

Az and X have the meaning given in Claim 1,

and their plant-tolerated acid addition salts and metal salt complexes.

3. Process for the preparation of 2-aryl-2-azolyl-methyl-1,3-dioxepines of the general formula (I)

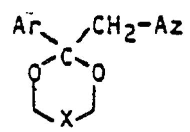

(I)

in which

Az represents imidazol-1-yl or 1,2,4-triazol-1-yl,

X represents the ethylene or vinylene grouping and

Ar represents phenyl, which can be optionally substituted by halogen, cyano, nitro, alkyl, alkoxy and alkylthio with in each case 1 to 4 carbon atoms, halogenoalkyl, halogenoalkoxy and halogenoalkylthio with in each case 1 to 2 carbon atoms and 1 to 5 halogen atoms, dialkylamino, alkoxycarbonyl, alkoxycarbonylamino and N-alkyl-alkoxycarbonylamino with in each case 1 to 4 carbon atoms in the particular alkyl parts, and by phenyl or phenoxy which are optionally substituted by halogen or alkyl with 1 to 4 carbon atoms,

and their plant-tolerated acid addition salts and metal salt complexes, characterised in that the corresponding 2-aryl-2-halogenomethyl-1,3-dioxepines of the formula (II)

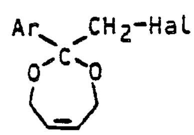

(II)

in which

Ar has the abovementioned meaning and

Hal represents halogen,

are reacted with alkali metal salts of azoles of the formula (III)

Az—M (III)

in which

Az has the abovementioned meaning and

M represents an alkali metal,

in the presence of a diluent, to give the compounds of the formula (Ia) according to the invention

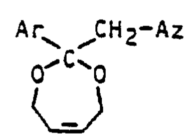

(Ia)

in which Ar and Az have the abovementioned meaning, and, if desired, these are then catalytically hydrogenated with hydrogen in the presence of a catalyst and if appropriate in the presence of a diluent to give the compounds of the formula (Ib) according to the invention

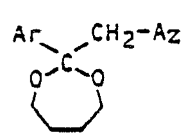

(Ib)

in which Ar and Az have the abovementioned meaning, and, if desired, an acid or a metal salt can then also be added on to the compounds of the formula (Ia) or (Ib) thus obtained.

4. Fungicidal agents, characterised in that they contain at least one 2-aryl-2-azolylmethyl-1,3-dioxepine of the formula (I) in Claim 1 and/or one of its plant tolerated acid addition salts and metal salt complexes.

5. Use of 2-aryl-2-azolylmethyl-1,3-dioxepines of the formula (I) in Claim 1 and/or their plant-tolerated acid addition salts and metal salt complexes as plant protection agents.

6. Use of 2-aryl-2-azolylmethyl-1,3-dioxepines of the formula (I) in Claim 1 and/or their plant-tolerated acid addition salts and metal salt complexes for combating fungi.

**Revendications**

1. 2-aryl-2-azolylméthyl-1,3-dioxépines de formule générale I

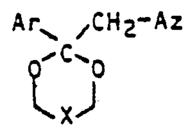

(I)

dans laquelle

Az représente un groupe imidazole-1-yle ou 1,2,4-triazole-1-yle,

X représente un groupement éthylène ou vinylène, et

Ar représente un groupe phényle éventuellement substitué par des halogènes, des groupes cyano, nitro, alkyle, alcoxy et alkylthio contenant chacun 1 à 4 atomes de carbone, halogénoalkyle, halogénoalcoxy et halogénoalkylthio contenant chacun 1 à 2 atomes de carbone et 1 à 5 atomes d'halogènes, dialkylamino, alcoxycarbonyle, alcoxycarbonylamino et N-alkyl-alcoxycarbonylamino contenant chacun 1 à 4 atomes de carbone dans chacune des parties alkyle, ou phényle ou phénoxy éventuellement substitué par des halogènes ou des groupes alkyle en $C_1$-$C_4$,

et leurs sels formés par addition avec des acides et complexes de sels métalliques tolérés par les végétaux.

2. Composés de formule I de la revendication 1, dans laquelle

Ar représente un groupe phényle portant éventuellement 1 à 3 substituants identiques ou différents choisis parmi le fluor, le chlore, le brome, les groupes cyano, nitro, méthyle, méthoxy, méthylthio, méthoxycarbonyle, trifluorométhyle, trifluorométhoxy, trifluorométhylthio, éthyle, éthoxy, éthoxycarbonyle, diméthylamino, acétamido, N-méthylacétamido, ou encore phényle ou phénoxy portant éventuellement 1 à 3 substituants identiques ou différents choisis parmi le fluor, le chlore ou les groupes méthyle, et

Az et X ont les sigifications indiquées dans la revendication 1,

ainsi que les sels formés par addition avec des acides et complexes de sels métalliques tolérés par les végétaux.

3. Procédé de préparation des 2-aryl-2-azolylméthyl-1,3-dioxépines de formule générale I

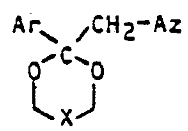

(I)

dans laquelle

Az représente un groupe imidazolyl-1-yle ou 1,2,4-triazole-1-yle,

X représente un groupement éthylène ou vinylène, et

Ar représente un groupe phényle éventuellement substitué par des halogènes, des groupes cyano, nitro, alkyle, alcoxy et alkylthio contenant chacun 1 à 4 atomes de carbone, halogénoalkyle, halogénoalcoxy et halogénoalkylthio contenant chacun 1 à 2 atomes de carbone et 1 à 5 atomes d'halogènes, dialkylamino, alcoxycarbonyle, alcoxycarbonylamino et N-alkyl-alcoxycarbonylamino contenant chacun 1 à 4 atomes de carbone dans chacune des parties alkyle, ou phényle ou phénoxy éventuellement substitué par des halogènes ou des groupes alkyle en $C_1$-$C_4$,

et de leurs sels formés par addition avec des acides et complexes de sels métalliques tolérés par les végétaux, caractérisé en ce que l'on fait réagir les 2-aryl-2-halogénométhyl-1,3-dioxépines correspondantes de formule II

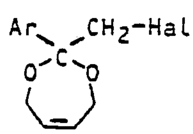

(II)

dans laquelle

Ar a les significations indiquées ci-dessus, et

Hal représente un halogène,

avec des sels alcalins d'azoles de formule III

$$Az—M \qquad (III)$$

dans laquelle

Az a les significations indiquées ci-dessus, et

M représente un métal alcalin,

en présence d'un diluant, la réaction donnant les composés selon l'invention répondant à la formule Ia

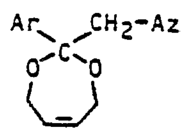

(Ia)

dans laquelle Ar et Az ont les significations indiquées ci-dessus, qu'on soumet éventuellement à hydrogénation en présence d'un catalyseur et éventuellement en présence d'un diluant, cette hydrogénation donnant les composés selon l'invention répondant à la formule Ib

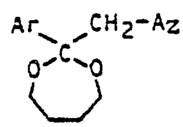

(Ib)

dans laquelle Ar et Az ont les significations indiquées ci-dessus, après quoi, le cas échéant, on fixe par addition un acide ou un sel métallique sur les composés de formule Ia ou Ib ainsi obtenus.

4. Produits fongicides caractérisés en ce qu'ils contiennent au moins une 2-aryl-2-azolylméthyl-1,3-dioxépine de formule I de la revendication 1 et/ou l'un de ses sels formés par addition avec des acides et complexes de sels métalliques tolérés par les végétaux.

5. Utilisation des 2-aryl-2-azolylméthyl-1,3-dioxépines de formule I de la revendication 1 et/ou de leurs sels formés par addition avec des acides et complexes de sels métalliques tolérés par les végétaux en tant qu'agents phytosanitaires.

6. Utilisation des 2-aryl-2-azolylméthyl-1,3-dioxépines de formule I de la revendication 1 et/ou de leurs sels formés par addition avec des acides et complexes de sels métalliques tolérés par les végétaux dans la lutte contre les mycètes.